# EUROPEAN PATENT SPECIFICATION

(11) **EP 3 400 905 B1**
(45) Date of publication and mention of the grant of the patent: **17.06.2020**
(21) Application number: 17870698.2
(22) Date of filing: 03.11.2017
(51) Int. Cl.: A61F 2/16

(54) **VARIABLE MULTIFOCAL ARTIFICIAL LENS**
VARIABLE MULTIFOKALE KÜNSTLICHE LINSE
LENTILLE ARTIFICIELLE MULTIFOCALE VARIABLE

(30) Priority: 16.11.2016 CN 201611008446
(43) Date of publication of application: 14.11.2018
(73) Proprietor: Vision Pro (Wuxi) Ltd, Wuxi, Jiangsu 214125 (CN)
(72) Inventor: LIAO, Xiugao, Wuxi Jiangsu 214125 (CN); FENG, Zhenyu, Wuxi Jiangsu 214125 (CN); YANG, Qin, Wuxi Jiangsu 214125 (CN)
(74) Representative: Hanna Moore + Curley
(86) International application number: PCT/CN2017/109292
(87) International publication number: WO 2018/090841

(56) References cited:
- WO-A1-99/29266
- WO-A2-2008/014496
- CN-A- 101 180 009
- CN-A- 101 528 156
- CN-A- 102 600 502
- CN-A- 106 667 623
- CN-U- 206 621 454
- US-A- 4 666 444
- US-A1- 2004 111 152
- US-A1- 2006 116 765
- US-A1- 2007 135 915

## Description

### TECHNICAL FIELD OF THE INVENTION

The present invention relates to the technical field of medical supplies, and in particular to a accommodative multifocal intraocular lens.

### BACKGROUND

The natural lens in the eye of a newborn baby is a colorless, transparency and very soft lens. As a person ages, the change of external conditions, such as ultraviolet radiation, the natural lens becomes more rigid and more color. When over fifty or sixty years, more than 30 percent of people's crystalline lens will turn yellow or brown or even cloudy. When this happens, not only lost the accommodative function, but also blurred vision and sensitivity to light.The cataractous lens will have to be replaced with an artificial eyesight of the cataract patient.

A typical intraocular lens is comprised of an optical lens and a supporting arm, particularly, the optical lens focuses light onto the optic nerve to enable the optical lens to see the object. The arm is used to support the optical zone, so that the optical zone of the optical lens in the center of the eye can be focused effectively.

The optical zone and supporting arm of the intraocular lens can be made of the same material or can be made of different materials. The intraocular lens made of the same material is commonly referred to as a one-piece lens, while the intraocular lens made of different materials is commonly referred to as a three-piece lens, and examples thereof are reported in U.S. Patent No. 4,997,442 and U.S. Patent No. 5,217,491, among these patents, the optical zones are both made of relatively soft optical materials and arm zone are both made of relatively hard materials.

The conventional monofocal intraocular lens can provide vision correction with a conventional distance, but cannot provide an effective accommodative vision correction. In other words, it cannot play a role of vision correction both at far and close distances. The only way to make the monofocal intraocular lens work both at far and close distances is to wear a pair of glasses. Another choice is to replace the cataract lens with a multifocal intraocular lens, to make vision at far, close and medium distances available. However, only a part of light at each distance is focused onto the optic nerve, in addition, multifocal intraocular lens will have some other side effects exist. As a result, people begin to design a novel accommodative-focus intraocular lens, as disclosed in U.S. Patent No. 4,409,691, U.S. Patent Nos. 5,674,282, 5,496,366, 6,197,059, and 6,387,126, U.S. Patent No. 6,178,878, and U.S. Patent No. 6,406,494.

All the designed accommodative intraocular lenses are made of a soft silicone material with a low refractive index. Due to the fact of lower the refractive index of the silicone material, the intraocular lens made from it, is relatively thick, the intraocular lens has a limited distance to move within the capsular bag of your eye, so that change in the accommodative-focus optical intensity is limited.

And also, the intraocular lens made from the silicone material will have a higher possibility to form fibers and secondary cataract than the intraocular lens made from hydrophobic polyacrylic ester material.

The relevant state of the art is represented by US 2004/111152 A1.

### SUMMARY

The present teaching provides an accommodative multifocal intraocular lens as detailed in claim 1. Advantageous features are provided in dependent claims.

In view of the problems existed in the prior art, the applicant provides a accommodative multifocal intraocular lens. The optical body of the present invention has a multifocal optical zone, and the back-and-forth movement of the optical zone can effectively increase the change of the focal power of the multifocal lens.

Technical solutions of the present invention are as follows:
A accommodative multifocal intraocular lens, including an optical body (1), a haptic (2) and a silicone connecting member (3), wherein the silicone connecting member (3) is located between the optical body (1) and the haptic (2), and connects the optical body (1) and the haptic (2) in an inserted manner through recesses and projections;
two ends of the optical body (1) are each provided with a recess (4); an end, joined to the optical body (1), of the haptic (2) is provided with a recess (5);
the optical zone of the optical body (1) is an optical zone having two focal points, three focal points, an infinite zoom region or regional multifocal points; and
two ends, along the width direction, of the silicone connecting member (3) are provided with projections, respectively, that is, a first projection (6) and a second projection (7), respectively.

The recesses and projections are rectangular or dovetail-shaped.

The intraocular lens is 11.5 to 13.5mm in diameter.

The optical body (1) is made of an optically transparent hydrophobic polyacrylic ester material having a refractive index of 1.48 to 1.56; the effective optical zone of the optical body (1) is 5.5 to 6.5 mm in diameter.

The haptic (2) is made of hydrophobic polyacrylic ester material or reinforced silicone material, and the haptic (2) is 0.18 to 0.65mm in thickness; the thickest portion of the silicone connecting member (3) is 0.18 to 0.65mm in thickness.

The recess (4) of the ends of optical body and the recess (5) of the end of the haptic are both 0.1 to 0.15mm in depth; the first projection (6) and the second projection (7) of the silicone connecting member (3) are both 0.1 to 0.15mm in height; the depths of the recess (4) of the ends of optical body and the recess (5) of the end of the haptic are consistent with the heights of the first (6) and the second (7) of the silicone connecting member (3).

The widths of the first projection (6) and the second projection (7) of the silicone connecting member (3) are consistent with the widths of the recess (4) of the ends of optical body and the recess (5) of the end of the haptic.

The material in the optical zone of the present invention is an optically transparent hydrophobic polyacrylate material, the material also can be acrylate, methacrylate or styrene derivative and small amounts of hydrophilic monomers, such as 2-hydroxyethyl methacrylate, 2-hydroxyethyl acrylate, 3-hydroxypropyl methacrylate, 3-hydroxypropyl acrylate, 4-hydroxybutyl methacrylate, 4-hydroxybutyl acrylate, N,N-dimethylacrylamide, N,N-dimethymethacrylamide, N,N-diethylacrylamide, N-ethylacrylamide.

The accommodative multifocal intraocular lens can first be made into an intraocular lens disk containing a multifocal optical surface, mechanically engraved into a one-piece lens, and then cut into an optical body and two haptics, and silicone connecting member is made by laminate molding.

The beneficial effects of the present invention lie in that:
The silicone connecting member adopted in the present invention is a a relatively soft material with good elasticity, when intraocular muscles contract and relax, the intraocular optical zone can be effectively moved back and forth.

The optical zone of the optical body in the present invention is an optical zone having two focal points, three focal points, an infinite zoom region or regional multifocal points; when the contraction or relaxation of the ciliary muscle, the original bifocal, trifocal, an infinite zoom region, or regional multifocal lens is changed into a new bifocal, trifocal, an infinite zoom region, or regional multifocal lens, and the optical power range will increase change in the optical power. Since the optical zone of the multifocal lens moves back and forth when the contraction or relaxation of the ciliary muscle, the original bifocal, trifocal, an infinite zoom region, or regional multifocal lens is changed into a new bifocal, trifocal, an infinite zoom region, or regional multifocal lens, and the optical power range will increase change in the optical power. As such, the adjustable optical power if the newly designed accommodative multifocal intraocular lens will be greater than the optical power range of any accommodative multifocal intraocular lens available in the market. Even if the lens loses the back-and-forth movement function for several reasons after several years, the lens will still maintain the original bifocal, trifocal, an infinite zoom region, or regional multifocal function.

When the accommodative multifocal intraocular lens moves back and forth, moving distances and optical power changes are as shown in table below:

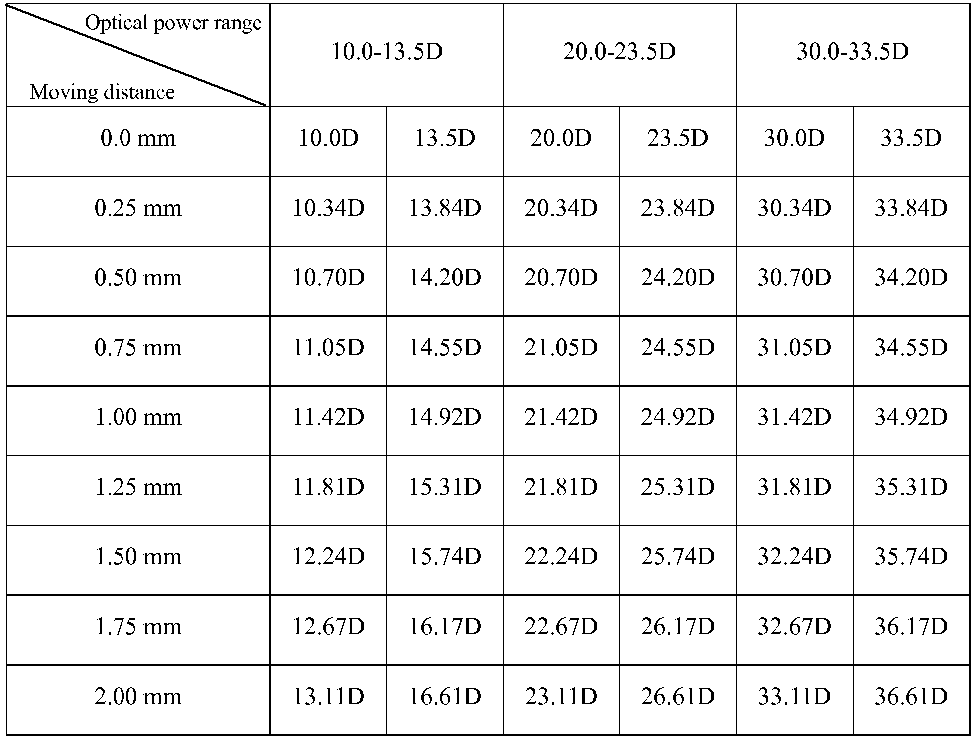

### BRIEF DESCRIPTION OF THE DRAWINGS

FIG. 1 is a shematic structural diagram according to the present invention;
FIG. 2 is a shematic structural diagram of an optical body according to the present invention;
FIG. 3 is a perspective schematic structural diagram of an optical body according to the present invention;
FIG. 4 is a shematic structural diagram of a haptic according to the present invention;
FIG. 5 is a perspective shematic structural diagram of a haptic according to the present invention;
FIG. 6 is a shematic structural diagram of a silicone connecting member according to the present invention;
FIG. 7 is a perspective shematic structural diagram of a silicone connecting member according to the present invention; and

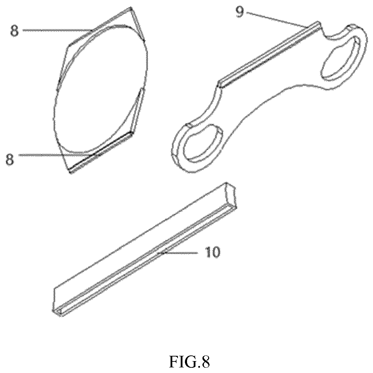

FIG. 8 is a shematic structural diagram according to an example.

In the figures: 1. optical body, 2. haptic, 3. silicone connecting member, 4. recess, 5. recess, 6. projection, 7. projection, 8. projection, 9. projection, 10. recess.

### DETAILED DESCRIPTION OF THE INVENTION

The present invention will be described in detail in conjunction with the accompanying drawings and embodiments.

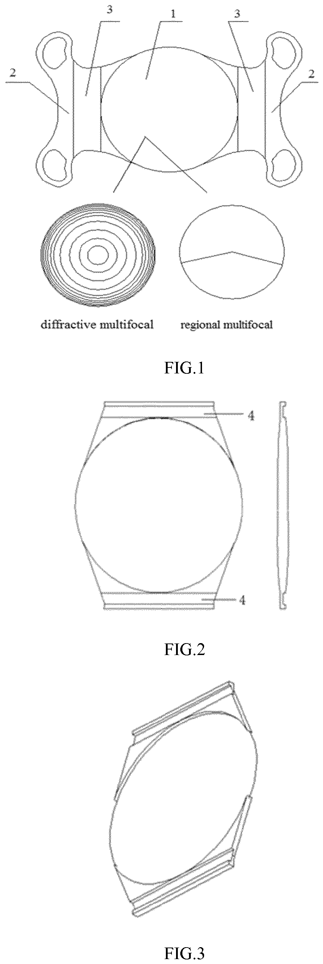

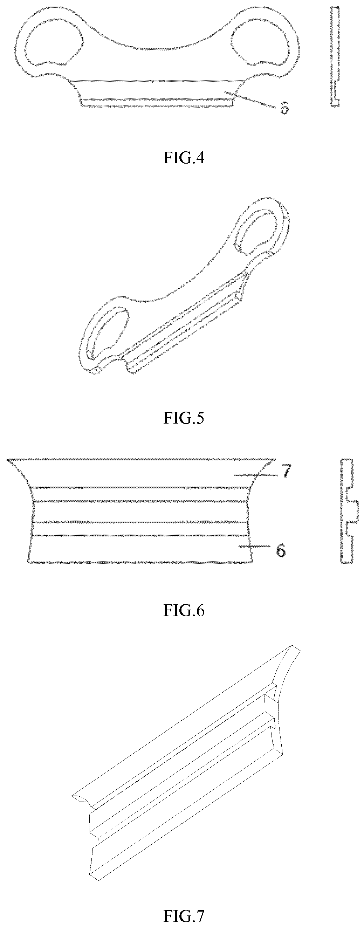

As shown in FIG. 1 to FIG. 7, a accommodative multifocal intraocular lens, including an optical body 1, a haptic 2 and a silicone connecting member 3, wherein the silicone connecting member 3 is located between the optical body 1 and the haptic 2, and connects the optical body 1 and the haptic 2 in an inserted manner through recesses and projections;

two ends of the optical body 1 are each provided with a recess 4; an end, joined to the optical body 1, of the haptic 2 is provided with a recess 5; the optical zone of the optical body 1 is an optical zone having two focal points, three focal points, an infinite zoom region or regional multifocal points;

two ends, along the width direction, of the silicone connecting member 3 are provided with projections, respectively, that is, a first projection 6 and a second projection 7, respectively.

The recesses and projections are rectangular or dovetail-shaped.

The intraocular lens is 11.5 to 13.5mm in diameter.

The optical body 1 is made of an optically transparent hydrophobic polyacrylic ester material having a refractive index of 1.48 to 1.56; the effective optical zone of the optical body 1 is 5.5 to 6.5 mm in diameter.

The haptic 2 is made of hydrophobic polyacrylic ester material or reinforced silicone material, and the haptic 2 is 0.18 to 0.65mm in thickness; the thickest portion of the silicone connecting member 3 is 0.18 to 0.65mm in thickness.

The recess 4 of the ends of optical body and the recess 5 of the end of the haptic are both 0.1 to 0.15mm in depth; the first projection 6 and the second projection 7 of the silicone connecting member are both 0.1 to 0.15mm in height; the depths of the recess 4 of the ends of optical body and the recess 5 of the end of the haptic are consistent with the heights of the first projection 6 and the second projection 7 of the silicone connecting member 3.

The widths of the first projection 6 and the second projection 7 of the silicone connecting member 3 are consistent with the widths of the recess 4 of the ends of optical body and the recess 5 of the end of the haptic.

When in use, the recesses and projections of two silicone connecting members 3 are applied with medical strong glue first, loaded on two ends of the optical body 1, and the projections at two ends of the optical body 1 are inserted into the recesses of the silicone connecting member 3, and correspondingly, a recesses at two ends of the optical body 1 coincide with the projections of the silicone connecting member 3, and then the projections at the other end of the two silicone connecting members 3 are loaded in the recesses of the two haptics 2, as such, the overall assembly of the three components is completed, and finally implanted into the capsular bag of the human eye.

### Embodiment 1

Referring to FIG. 1 to FIG. 7, an accommodative trifocal intraocular lens is an assembled intraocular lens with three focal points of 13.5D, 20.0D, 23.5D, respectively, and the preparation method thereof is:
(1) an intraocular lens disk (13mm in diameter) containing a trifocal optical surface can first be made;
(2) the disk made in step (1) is mechanically engraved into a one-piece lens, and then cut into an optical body and two haptics, and silicone connecting member is made by laminate molding;
(3) recesses and projections of two silicone connecting members are applied with medical strong glue first, loaded on two ends of the optical body, and the projections at two ends of the optical body are inserted into the recesses of the silicone connecting member, and correspondingly, recesses at two ends of the optical body coincide with the projections of the silicone connecting member, and then the projections at the other end of the two silicone connecting members are loaded in the recesses of the two haptics, as such, the overall assembly of the three components is completed, and finally implanted into the capsular bag of the human eye;
   the optical body is made of a hydrophobic polyacrylic ester material and the effective optical zone of the optical body is 5.5 mm in diameter and 0.6±0.2mm in thickness;
   the haptic is made of hydrophobic polyacrylic ester material and the haptic is 0.3±0.1mm in thickness;
   the overall width of the silicone connecting member is 1.5mm and the thickness in the center is 0.3mm; the projection in the middle of the silicone connecting member is 0.3mm in width, projections at two ends are both 0.4mm in width, and recesses in the middle are both 0.2mm in width.

When the contraction or relaxation of the ciliary muscle, the back-and-forth moving distance is 1.5mm, and the zoom regions of the intraocular lens in the present invention are in ranges of 13.5D-15.74D, 20.0D-22.24D and 23.5D-25.74D.

### Embodiment 2

Referring to FIG. 1 to FIG. 7, an accommodative trifocal intraocular lens is an assembled intraocular lens with three focal points of 10.0D, 20.0D, 30.0D, respectively, and the preparation method thereof is:
(1) an intraocular lens disk (13mm in diameter) containing a trifocal optical surface can first be made;
(2) the disk made in step (1) is mechanically engraved into a one-piece lens, and then cut into an optical body and two haptics, and silicone connecting member is made by laminate molding;
(3) projections and recesses of two silicone connecting members are applied with medical strong glue first, loaded on two ends of the optical body, and the projections at two ends of the optical body are inserted into the recesses of the silicone connecting member, and correspondingly, recesses at two ends of the optical body coincide with the projections of the silicone connecting member, and then the projections at the other end of the two silicone connecting members are loaded in the recesses of the two haptics, as such, the overall assembly of the three components is completed, and finally implanted into the capsular bag of the human eye;
   the optical body is made of a hydrophobic polyacrylic ester material and the effective optical zone of the optical body is 6 mm in diameter and 0.6±0.2mm in thickness;
   the haptic is made of hydrophobic polyacrylic ester material and the haptic is 0.3±0.1mm in thickness;
   the overall width of the silicone connecting member is 1.2mm and the thickness in the center is 0.3mm; the projection in the middle of the silicone connecting member is 0.1mm in width, projections at two ends are both 0.4mm in width, and recesses in the middle are both 0.15mm in width.

When the contraction or relaxation of the ciliary muscle, the back-and-forth moving distance is 1.25mm, and the zoom regions of the intraocular lens are in ranges of 10.0D-11.81D, 20.0D-21.81D and 30.0D-31.81D;

## Claims

1. An accommodative multifocal intraocular lens, comprising an optical body (1), two haptics (2) and two silicone connecting members (3), the silicone connecting members (3) being located between the optical body (1) and the haptics (2), and connecting the optical body (1) and the haptics (2) in an inserted manner through recesses and projections;
wherein two ends along a width of each silicone connecting member (3) are each provided with projections (6, 7);
wherein two ends of the optical body (1) are each provided with a recess (4);
wherein an end, joined to the optical body (1), of each haptic (2) is provided with a recess (5); wherein the projections (6, 7) of each silicone connecting member (3) are inserted into the corresponding recesses of the optical body (1) and the haptic (2);
wherein the silicone connecting members (3) are connected to the optical body (1) and the haptics (2) by means of glue; and wherein the optical zone of the optical body (1) is an optical zone having two focal points, three focal points, an infinite zoom region or regional multifocal points.

2. The accommodative multifocal intraocular lens according to claim 1, wherein, the recesses and projections are rectangular or dovetail-shaped.

3. The accommodative multifocal intraocular lens according to claim 1, wherein, the intraocular lens is 11.5 to 13.5mm in diameter.

4. The accommodative multifocal intraocular lens according to claim 1, wherein, the optical body (1) is made of an optically transparent hydrophobic polyacrylic ester material having a refractive index of 1.48 to 1.56; the effective optical zone of the optical body (1) is 5.5 to 6.5 mm in diameter.

5. The accommodative multifocal intraocular lens according to claim 1, wherein, the haptic (2) is made of hydrophobic polyacrylic ester material or reinforced silicone material, and the haptic (2) is 0.18 to 0.65mm in thickness; the thickest portion of the silicone connecting member (3) is 0.18 to 0.65mm in thickness.

6. The accommodative multifocal intraocular lens according to claim 1, wherein, the recess (4) of the ends of optical body and the recess (5) of the end of the haptic are both 0.1 to 0.15mm in depth; the first projection (6) and the second projection (7) of the silicone connecting member (3) are both 0.1 to 0.15mm in height; the depths of the recess (4) of the ends of optical body and the recess (5) of the end of the haptic are consistent with the heights of the first projection (6) and the second projection (7) of the silicone connecting member (3).

7. The accommodative multifocal intraocular lens according to claim 1, wherein, the widths of the first projection (6) and the second projection (7) of the silicone connecting member (3) are consistent with the widths of the recess (4) of the ends of optical body and the recess (5) of the end of the haptic.

## Patentansprüche

1. Akkommodierende multifokale Intraokularlinse, umfassend einen optischen Körper (1), zwei Haptiken (2) und zwei Silikonverbindungselemente (3), wobei die Silikonverbindungselemente (3) sich zwischen dem optischen Körper (1) und den Haptiken (2) befinden und den optischen Körper (1) und die Haptiken (2) in einer eingeführten Weise durch Aussparungen und Vorsprünge verbinden;
wobei zwei Enden entlang einer Breite jedes Silikonverbindungselements (3) jeweils mit Vorsprüngen (6, 7) versehen sind;
wobei zwei Enden des optischen Körpers (1) jeweils mit einer Aussparung (4) versehen sind;
wobei ein mit dem optischen Körper (1) verbundenes Ende jeder Haptik (2) mit einer Aussparung (5) versehen ist;
wobei die Vorsprünge (6, 7) jedes Silikonverbindungselements (3) in die entsprechenden Aussparungen des optischen Körpers (1) und der Haptik (2) eingeführt sind;
wobei die Silikonverbindungselemente (3) mittels Klebstoff mit dem optischen Körper (1) und der Haptik (2) verbunden sind; und
wobei die optische Zone des optischen Körpers (1) eine optische Zone mit zwei Brennpunkten, drei Brennpunkten, einem unendlichen Zoombereich oder regionalen multifokalen Punkten ist.

2. Akkommodierende multifokale Intraokularlinse nach Anspruch 1, wobei die Aussparungen und Vorsprünge rechteckig oder schwalbenschwanzförmig sind.

3. Akkommodierende multifokale Intraokularlinse nach Anspruch 1, wobei die Intraokularlinse 11,5 bis 13,5 mm im Durchmesser ist.

4. Akkommodierende multifokale Intraokularlinse nach Anspruch 1, wobei der optische Körper (1) aus einem optisch transparenten hydrophoben Polyacrylestermaterial mit einem Brechungsindex von 1,48 bis 1,56 hergestellt ist; wobei die wirksame optische Zone des optischen Körpers (1) 5,5 bis 6,5 mm im Durchmesser ist.

5. Akkommodierende multifokale Intraokularlinse nach Anspruch 1, wobei die Haptik (2) aus hydrophobem Polyacrylestermaterial oder verstärktem Silikonmaterial hergestellt ist und die Haptik (2) 0,18 bis 0,65 mm in der Dicke ist; wobei der dickste Abschnitt des Silikonverbindungselements (3) 0,18 bis 0,65 mm in der Dicke ist.

6. Akkommodierende multifokale Intraokularlinse nach Anspruch 1, wobei die Aussparung (4) der Enden des optischen Körpers und die Aussparung (5) des Endes der Haptik beide 0,1 bis 0,15 mm in der Tiefe sind; wobei der erste Vorsprung (6) und der zweite Vorsprung (7) des Silikonverbindungselements (3) beide 0,1 bis 0,15 mm in der Höhe sind; wobei die Tiefen der Aussparung (4) der Enden des optischen Körpers und der Aussparung (5) des Endes der Haptik mit den Höhen des ersten Vorsprungs (6) und des zweiten Vorsprungs (7) des Silikonverbindungselements (3) übereinstimmen.

7. Akkommodierende multifokale Intraokularlinse nach Anspruch 1, wobei die Breiten des ersten Vorsprungs (6) und des zweiten Vorsprungs (7) des Silikonverbindungselements (3) mit den Breiten der Aussparung (4) der Enden des optischen Körpers und der Aussparung (5) des Endes der Haptik übereinstimmen.

## Revendications

1. Lentille intraoculaire multifocale accommodative, comprenant un corps optique (1), deux haptiques (2) et deux éléments de liaison en silicone (3), les éléments de liaison en silicone (3) étant situés entre le corps optique (1) et les haptiques (2), et reliant le corps optique (1) et les haptiques (2) d'une manière insérée au travers d'évidements et de saillies ;
deux extrémités le long d'une largeur de chaque élément de liaison en silicone (3) étant chacune pourvues de saillies (6, 7) ;
deux extrémités du corps optique (1) étant chacune pourvues d'un évidement (4) ; une extrémité, jointe au corps optique (1), de chaque haptique (2) étant pourvue d'un évidement (5) ;
les saillies (6, 7) de chaque élément de liaison en silicone (3) étant insérées dans les évidements correspondants du corps optique (1) et de l'haptique (2) ;
les éléments de liaison en silicone (3) étant reliés au corps optique (1) et aux haptiques (2) au moyen de colle ; et
la zone optique du corps optique (1) étant une zone optique ayant deux points focaux, trois points focaux, une région de zoom infinie ou des points multifocaux régionaux.

2. Lentille intraoculaire multifocale accommodative selon la revendication 1, les évidements et les saillies étant rectangulaires ou en forme de queue d'aronde.

3. Lentille intraoculaire multifocale accommodative selon la revendication 1, la lentille intraoculaire ayant un diamètre de 11,5 à 13,5 mm.

4. Lentille intraoculaire multifocale accommodative selon la revendication 1, le corps optique (1) étant composé d'un ester polyacrylique hydrophobe optiquement transparent ayant un indice de réfraction de 1,48 à 1,56 ; la zone optique effective du corps optique (1) ayant un diamètre de 5,5 à 6,5 mm.

5. Lentille intraoculaire multifocale accommodative selon la revendication 1, l'haptique (2) étant composé d'un ester polyacrylique hydrophobe ou d'un matériau silicone renforcé, et l'haptique (2) ayant une épaisseur de 0,18 à 0,65 mm ; la partie la plus épaisse de l'élément de liaison en silicone (3) ayant une épaisseur de 0,18 à 0,65 mm.

6. Lentille intraoculaire multifocale accommodative selon la revendication 1, l'évidement (4) des extrémités du corps optique et l'évidement (5) de l'extrémité de l'haptique ayant tous deux une profondeur de 0,1 à 0,15 mm ; la première saillie (6) et la seconde saillie (7) de l'élément de liaison en silicone (3) ayant toutes deux une hauteur de 0,1 à 0,15 mm ; les profondeurs de l'évidement (4) des extrémités du corps optique et de l'évidement (5) de l'extrémité de l'haptique étant cohérentes avec les hauteurs de la première saillie (6) et de la seconde saillie (7) de l'élément de liaison en silicone (3).

7. Lentille intraoculaire multifocale accommodative selon la revendication 1, les largeurs de la première saillie (6) et de la seconde saillie (7) de l'élément de liaison en silicone (3) étant cohérentes avec les largeurs de l'évidement (4) des extrémités du corps optique et de l'évidement (5) de l'extrémité de l'haptique.
